# NEW EUROPEAN PATENT SPECIFICATION

(11) **EP 1 750 628 B2**
(45) Date of publication and mention of the opposition decision: **28.08.2013**
(45) Mention of the grant of the patent: 03.03.2010
(21) Application number: 05744183.4
(22) Date of filing: 19.05.2005
(51) Int. Cl.: A61F 5/44

(54) **OSTOMY POUCHES**
OSTOMIE-BEUTEL
POCHES DE STOMIE

(30) Priority: 04.06.2004 GB 0412489; 20.10.2004 GB 0423285
(43) Date of publication of application: 14.02.2007
(73) Proprietor: WELLAND MEDICAL LIMITED, Crawley, West Sussex RH10 2TU (GB)
(72) Inventor: SMITH, Rory, Crawley, West Sussex RH10 2TU (GB); MAY, Owen, Crawley, West Sussex RH10 2TU (GB); BIRD, Paul, Crawley, West Sussex RH10 2TU (GB)
(74) Representative: Lock, Graham James
(86) International application number: PCT/GB2005/001956
(87) International publication number: WO 2005/117775

(56) References cited:
- EP-A- 1 378 218
- WO-A-99/66859
- WO-A1-03//065944
- WO-A1-03//086250
- WO-A1-99//025278
- US-B1- 3 189 253
- Summary Curriculum Vitae Dr. Rory Smith

## Description

The present invention relates to ostomy pouches and drainage pouches, and in particular to sealing mechanisms for such pouches which operate to retain the pouch contents during use allowing disposal of the contents more readily and hygienically after use. The document WO 03/086250 is regarded as the closest prior art.

By "pouch" it is meant the inner pouch of an ostomy bag, which receives and carries bodily waste.

Ostomy pouches for receiving bodily waste from colostomy or ileostomy patients are well known and a problem with such pouches is the opening/closing mechanism of the pouch. Such a mechanism must provide a good seal when the discharge opening is closed in use, to prevent leakage of the contents of the bag, while also ensuring an easy and efficient way of opening the discharge opening of the bag for disposal of the contents.

It is known, for example in WO 03/065944 A1, to provide ostomy bags with a neck portion which includes a discharge opening, the neck portion being closed by folding the neck portion upwardly and opened by unfolding the neck downwardly. The neck portion is made from, for example, a polymeric film.

WO 03/065944 A1 also discusses transversely extending bias members formed of stiff but flexible material on each side wall of the neck portion. The discharge opening is opened by applying inwardly-directed finger pressure to the opposite ends of the bias members to cause them to bow outwardly away from each other.

The present applicant's have found several disadvantages with this, and other prior art mechanisms. Firstly, when the neck portion is closed by folding, the first fold (folding the bias member upwardly against the neck material) does not provide a tight seal as the fold provided by the neck material is not particularly sharp. As a result, the contents of the ostomy bag can often leak into and through the folds in the neck portion when the bag is closed.

The present invention sets out to provide an ostomy bag with an improved opening/closing mechanism that overcomes or at least alleviates the problems described above.

In one aspect, the invention provides a drainable pouch for an ostomy bag as defined in the appended claims.

Preferably, during folding of the neck portion in use, the first and second members are initially folded with respect to each other such that the first and second members are located either side of the fold.

Preferably, the first and second members are of equal length.

Preferably, the discharge opening is openable by outwardly directed pressure applied to the tab of the neck portion.

The invention will now be described by way of example with reference to the accompanying diagrammatic drawings, in which :-
Figure 1 is a plan view showing one side of an ostomy pouch constructed in accordance with the present invention;
Figure 2 is a plan view showing the other side of the ostomy pouch of Figure 1 in accordance with the present invention; and
Figure 3 is an end view of the ostomy pouch of Figures 1 and 2 showing the discharge opening in a fully opened condition caused by outwardly directed pressure applied to a tab of the neck portion; and

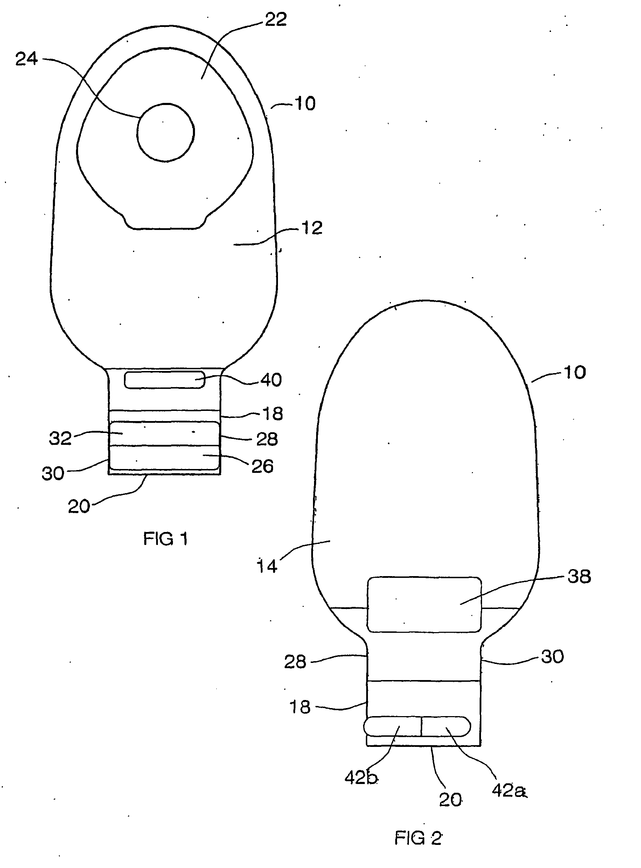

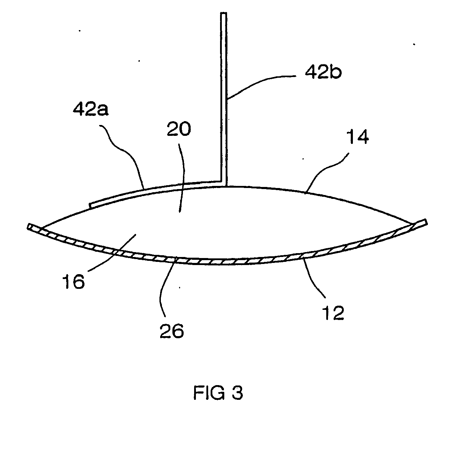

Referring first to Figures 1 and 2, a drainable ostomy pouch 10 has two generally parallel side walls 12, 14 of polymeric film or similar material suitable for an ostomy pouch or the like. The side walls 12, 14 are joined along their edges to provide a cavity 16 therebetween (best seen Figures 3 and 5) in which bodily waste can be contained. Part of the side walls 12, 14 define a neck portion 18 which terminates in a discharge opening 20 (also best seen in Figures 3 and 5) to allow drainage of the contents of the pouch 10 when the discharge opening 20 is in its open condition.

Figure 1 shows a first side wall 12 of the ostomy pouch 10. On this wall 12, a first transversely-extending elongate strip 26 is located at the end of the neck portion 18 of the pouch 10. The strip 26 is flat when the discharge opening 20 is in its closed condition. The strip 26 is made of a stiff but resiliently flexible plastics material.

The first strip 26 extends across the entire lateral length of the neck portion 18 between the side edges 28, 30 of the neck portion 18.

A second transversely-extending elongate strip 32 is located adjacent and parallel to the first strip 26. The second strip 32 is of the same material as the first strip 26 and is flat when the discharge opening 20 is in its closed condition. Like the first strip 26, the second strip 32 extends across the entire lateral length of the neck portion 18 between the side edges 28, 30 of the neck portion 18.

A plate 22 of a skin friendly adhesive barrier material is attached to the side wall 12. The plate 22 serves to attach the pouch 10 to the skin surface of a wearer of the ostomy pouch 10 in a manner well known in the art. Both the side wall 12 and the plate 22 have apertures 24 for receiving a stoma, in a manner which is also well known in the art.

Figure 2 shows a second side wall 14 of the ostomy pouch. A retaining strip 38 is provided on the side wall 14 at the base of the neck portion 18. The retaining strip 38 is designed to engage with a complimentary retaining strip 40 located on the other side wall 12 of the pouch (shown in Figure 1). The two retaining strips 38, 40 may, for example, be complimentary halves of a strip of VELCRO^{™} or the like. The way in which the strips 38, 40 engage each will be described later.

Figure 2 also shows a tab 42 on the second side wall 14 of the ostomy pouch. The tab 42 extends outwardly from the side wall 14 at a point generally equidistant from the ends of the neck portion 18. The tab 42 is made from a non-stiff material such as polymeric film. A first half of the tab 42a is attached to the sidewall and lies flat next to the first strip 26 when the discharge opening 20 is in its closed condition. This first half of the tab 42a is not resilient and simply acts to secure the second half of the tab 42b to the neck portion 18.

The initial folding of the neck portion 18 involves folding the first strip 26 and the tab 42 upwards against the second strip 32. In this way, the first and second strips 26, 32 lie against each other either side of the fold (with the tab 42 inside the fold). The fact that the fold has a strip 26, 32 either side of the fold ensures a sharp fold through which the contents cannot pass thus totally sealing the opening and preventing leakage. To close the neck, the rest of the neck portion 18 is continually folded upwardly until the first and second retaining strips 38, 40 on either side wall 12, 14 engage and attach to one another to retain the neck portion 18 in a folded position.

When the neck portion 18 is unfolded and the strips 26, 32 lie flat, the discharge opening 20 still needs to be opened fully before the contents of the pouch 10 can be drained. The discharge opening 20 is fully opened by applying outwardly directed pressure (away from the plane of the transversely extending strip) to the tab 42b of the neck portion. This can be seen clearly in Figure 3. The applied pressure will cause the side walls of the neck portion 12, 14 to be pulled apart, thereby allowing drainage of the contents of the pouch 10.

## Claims

1. A drainable pouch (10) for an ostomy bag, having first (12) and second (14) side walls forming a cavity (16) therebetween, the side walls defining a foldable neck portion (18) terminating in a discharge opening (20) for draining contents of the pouch (10) from the cavity (16), the discharge opening (20) being closed and opened by folding and unfolding the neck portion (18) respectively, the first side wall (12) of the neck portion having a first transversely-extending resiliently flexible member (26) adjacent the discharge opening (20) wherein a second transversely-extending resiliently flexible member (32) is on said first side wall adjacent the first flexible member (26) and wherein the second side wall (14) of the neck portion (18) has a tab (42) attached thereto and wherein at least part of a tab (42) extends outwardly from the second side wall (14) at a point generally equidistant from the ends of the neck portion (18) and **characterised in that** the tab is an elongate strip of polymeric film arranged in parallel alignment with the second transversely-extending resiliently flexible member (32) when the discharge opening (20) is closed, a first half (42a) of which is attached to the second side wall (14) and lies flat next to the first resiliently flexible member (26) when the discharge opening (20) is in its closed condition and the second half (42b) of which extends from the side wall from the point generally equidistant from the ends of the neck portion (18).

2. A drainable pouch (10) according to claim 1, wherein, during folding of the neck portion (18) in use, the first and second members (26, 32) are initially folded with respect to each other such that the first and second members (26, 32) are located either side of the fold.

3. A drainable pouch (10) according to claim 1 to claim 2, the first and second members (26, 32) are of equal length.

4. A drainable pouch (10) according to any preceding claim, wherein the discharge opening (20) is openable by outwardly directed pressure applied to the tab (42) of the neck portion (18).

## Patentansprüche

1. Entwässerbarer Beutel (10) für einen Ostomie-Beutel aufweisend eine erste (12) und eine zweite (14) Seitenwand, die dazwischen einen Hohlraum bilden, wobei die Seitenwände einen faltbaren Verengungsbereich (18) bilden, der eine Ausflussöffnung (20) zum Ablassen des Inhalts des Beutels (10) aus dem Hohlraum (16) begrenzt, wobei die Ausflussöffnung (20) durch Falten und Entfalten jeweils geöffnet oder geschlossen wird, wobei die erste Seitenwand (12) des Verengungsbereichs (18) ein erstes sich schräg erstreckendes, belastbar flexibles Teil (26) aufweist, das an die Ausflussöffnung (20) angrenzt, wobei ein zweites sich schräg erstreckendes, belastbar flexibles Teil (32) sich an besagter erster Seitenwand, angrenzend an das erste flexible Teil (26) befindet und wobei die zweite Seitenwand (14) des Verengungsbereichs (18) einen daran angebrachten Streifen (42) aufweist und wobei sich wenigstens ein Teil des Streifens (42) nach außen von der zweiten Seitenwand (14) an einem Punkt erstreckt, der im Wesentlichen gleich weit von den Enden des Verengungsbereichs (18) entfernt ist und **dadurch gekennzeichnet ist, dass** der Streifen ein länglicher schmaler Bandstreifen polimerischen Films ist, angeordnet in paralleler Ausrichtung zu dem zweiten sich schräg erstreckenden, belastbar flexiblen Teil (32), wenn die Ausflussöffnung (20) geschlossen ist, wobei eine erste Hälfte (42a) davon an der zweiten Seitenwand (14) angebracht ist, und flach neben dem ersten Streifen (26) liegt, wenn die Ausflussöffnung (20) sich in ihrem geschlossenen Zustand befindet, und die zweite Hälfte (42b) davon sich von der Seitenwand von dem Punkt, der im Wesentlichen gleich weit von den Enden des Verengungsbereichs (16) entfernt ist, erstreckt.

2. Entwässerbarer Beutel (10) nach Anspruch 1, wobei während des Faltens des Verengungsbereiches (18) bei der Verwendung das erste und das zweite Teil (26, 32) zunächst zueinander so gefaltet werden, dass das erste und das zweite Teil (26, 32) auf beiden Seiten der Falte angeordnet sind.

3. Entwässerbarer Beutel (10) nach Anspruch 1 oder 2, wobei das erste und das zweite Teil (26, 32) von gleicher Länge sind.

4. Entwässerbarer Beutel (10) nach einem der vorangehenden Ansprüche, wobei die Ausflussöffnung (20) durch nach außen gerichteten Druck geöffnet werden kann, der an den Streifen (42) des Verengungsbereichs (18) angelegt wird.

## Revendications

1. Poche pouvant être vidée (10) pour une poche stomique, possédant des première (12) et seconde (14) parois latérales formant une cavité (16) entre celles-ci, les parois latérales définissant une partie de col pliante (18) se terminant en une ouverture d'évacuation (20) pour vider le contenu de la poche (10) à partir de la cavité (16), l'ouverture d'évacuation (20) étant fermée et ouverte en pliant et dépliant la partie de col (18) respectivement, la première paroi latérale (12) de la partie de col possédant un premier élément s'étendant transversalement et flexible de façon élastique (26) adjacent à l'ouverture d'évacuation (20), dans laquelle un deuxième élément s'étendant transversalement et flexible de façon élastique (32) se trouve sur ladite première paroi latérale adjacent au premier élément flexible (26) et dans laquelle la seconde paroi latérale (14) de la partie de col (18) comporte une languette (42) fixée à celle-ci et dans laquelle au moins une partie d'une languette (42) s'étend vers l'extérieur à partir de la seconde paroi latérale (14) à un point généralement équidistant des extrémités de la partie de col (18) et **caractérisée en ce que** la languette est une bande allongée de film polymère disposée selon un alignement parallèle avec le deuxième élément s'étendant transversalement et flexible de façon élastique (32) lorsque l'ouverture d'évacuation (20) est fermée, une première moitié (42a) de celle-ci est fixée à la seconde paroi latérale (14) et se trouve à plat à côté de la première bande (26) lorsque l'ouverture d'évacuation (20) est dans sa condition fermée, et la seconde moitié (42b) de celle-ci s'étend à partir la paroi latérale depuis le point généralement équidistant des extrémités de la partie de col (16).

2. Poche pouvant être vidée (10) selon la revendication 1, dans laquelle, au cours du pliage de la partie de col (18) durant l'utilisation, les premier et deuxième éléments (26, 32) sont initialement pliés l'un par rapport à l'autre de sorte que les premier et deuxième éléments (26, 32) soient situés de chaque côté du pli.

3. Poche pouvant être vidée (10) selon la revendication 1 ou la revendication 2, dans laquelle les premier et deuxième éléments (26, 32) sont de longueur égale.

4. Poche pouvant être vidée (10) selon l'une quelconque des revendications précédentes, dans laquelle l'ouverture d'évacuation (20) peut être ouverte par une pression dirigée vers l'extérieur appliquée sur la languette (42) de la partie de col (18).
